# EUROPEAN PATENT APPLICATION

(11) **EP 3 566 635 A1**
(43) Date of publication of application: **13.11.2019**
(21) Application number: 17889985.2
(22) Date of filing: 24.11.2017
(51) Int. Cl.: A61B 1/00, A61B 1/045, A61B 1/06, G02B 23/24, G06T 7/00, H04N 5/225, H04N 7/18

(54) **ENDOSCOPE DEVICE AND IMAGE GENERATION METHOD FOR ENDOSCOPE DEVICE**

(30) Priority: 04.01.2017 JP 2017000117
(71) Applicant: Sony Corporation, Tokyo 108-0075 (JP)
(72) Inventor: TSURU, Daisuke, Tokyo 108-0075 (JP); TAKAHASHI, Kenji, Tokyo 108-0075 (JP); TAKAHASHI, Yasuaki, Tokyo 108-0075 (JP); SUGIE, Yuki, Tokyo 108-0075 (JP); FUKAZAWA, Kentaro, Tokyo 108-0075 (JP)
(74) Representative: D Young & Co LLP
(86) International application number: PCT/JP2017/042293
(87) International publication number: WO 2018/128028

(57) **Abstract**

[Object] To simultaneously meet both of acquisition of depth information with high resolution and high accuracy and a reduction in diameter of a leading end of an endoscope.

[Solving Means] Provided is an endoscope apparatus including: an irradiation unit that irradiates a subject with illumination light and with a structured pattern for acquiring depth information of the subject; and an acquisition unit that acquires reflected light of the light emitted by the irradiation unit. With this configuration, it is possible to simultaneously meet both of acquisition of depth information with high resolution and high accuracy and a reduction in diameter of a leading end of an endoscope.

## Description

### Technical Field

The present disclosure relates to an endoscope apparatus and an image generation method for an endoscope apparatus.

### Background Art

In the past, for example, Patent Literature 1 mentioned below describes an endoscope apparatus including a light source lamp for supplying illumination light, light transmission means for transmitting the illumination light, and an imaging device that captures an image of a subject illuminated with the illumination light transmitted by the light transmission means.

### Citation List

### Patent Literature

Patent Literature 1: Japanese Patent Application Laid-open No. 2002-119468

### Disclosure of Invention

### Technical Problem

In an endoscope image, when depth information can be acquired in addition to an observation image,
the depth information is applicable to many applications and becomes more useful as the accuracy and resolution thereof become higher. The depth information is conceived to have many applications to, for example, segmentation/registration of an organ or object recognition. As the ranging technology of acquiring the depth information, there are a TOF (Time of Flight) method, a binocular parallax method, an SLAM (Simultaneous Localization and mapping) method, a structured light method, and the like. However, in the TOF method, the resolution of TOF is a VGA level, and a high-definition image cannot be obtained. The binocular parallax method needs two lens holes at the leading end of a rigid scope, which makes it difficult to reduce the diameter thereof. If the lens diameter is intended to be reduced, drawbacks in the resolution or image quality are caused. In the SLAM, it is assumed that a subject or a camera remains still, and within a living body, illumination conditions on the motion of a flexible object, a bright spot, and the like are strict, which makes it difficult to follow a change in feature point between images. In the structured light method, because it is necessary to provide independent illumination windows for normal illumination and illumination for structured light at the leading end of the leading end of a rigid scope, the diameter is difficult to reduce. In addition, because the normal illumination and the structured illumination are switched in a time division manner, in order to acquire a structured image, a frame rate of the normal image becomes half, which is problematic.

In recent healthcare, the needs of low invasiveness have been increasing. Because of the influence on postoperative QoL, there is a demand for minimization of an incised wound. Therefore, there is a demand for further reduction in diameter of an endoscope to be inserted into the inside of a human body.

The technology described in Patent Literature 1 described above relates to an endoscope apparatus including an imaging device that captures an image of a subject illuminated with illumination light transmitted by light transmission means. When depth information is intended to be acquired, in order to acquire the depth information, it is necessary to provide an irradiation window that irradiates the subject with illumination, and it becomes difficult to reduce the diameter of the leading end of the endoscope. As in a stereoscopic image, there is also a method of acquiring images of a plurality of viewpoints to acquire depth information. However, also in this case, in order to acquire images of a plurality of viewpoints, reducing the diameter of the leading end of the endoscope becomes difficult.

In this regard, there has been a demand for simultaneously meeting both of acquisition of depth information with high resolution and high accuracy and a reduction in diameter of a leading end of an endoscope.

### Solution to Problem

According to the present disclosure, there is provided an endoscope apparatus including: an irradiation unit that irradiates a subject with illumination light and with a structured pattern for acquiring depth information of the subject; and an acquisition unit that acquires reflected light of the light emitted by the irradiation unit.

Further, according to the present disclosure, there is provided an image generation method for an endoscope apparatus, the endoscope apparatus including an irradiation unit that irradiates a subject with illumination light and with a structured pattern for acquiring depth information of the subject, and an acquisition unit that acquires reflected light of the light emitted by the irradiation unit, the method including: capturing an image of the reflected light and acquiring a captured image; and separating structured pixels of the structured pattern from the captured image and generating an observation image. Advantageous Effects of Invention

According to the present disclosure, it is possible to simultaneously meet both of acquisition of depth information with high resolution and high accuracy and a reduction in diameter of a leading end of an endoscope.

It should be noted that the effects described above are not necessarily limitative. With or in the place of the above effects, any of the effects described in this specification or other effects that may be grasped from this specification may be achieved.

### Brief Description of Drawings

[Fig. 1] Fig. 1 is a schematic view showing a configuration of a system according to an embodiment of the present disclosure.
[Fig. 2] Fig. 2 is a schematic view showing a leading end of a rigid scope.
[Fig. 3] Fig. 3 is a schematic view showing a method of irradiating a subject with a structured pattern and estimating the structure of the subject (distance information) from a change in structured pattern on the subject.
[Fig. 4A] Fig. 4A is a schematic view showing an example of providing both of a normal illumination window and an illumination window for structured light to a leading end of a rigid scope 100.
[Fig. 4B] Fig. 4B is a schematic view showing an example of providing two camera windows to the leading end of the rigid scope so as to acquire right and left images.
[Fig. 5] Fig. 5 is a schematic view showing an irradiation pattern in this embodiment.
[Fig. 6] Fig. 6 is a schematic view showing a technique of separating a ranging image and an observation image from a captured image.
[Fig. 7] Fig. 7 is a flowchart showing processing of separating a ranging image and an observation image from each other on the basis of Fig. 6.
[Fig. 8A] Fig. 8A is a schematic view showing a crosshatch pattern characteristic of a superficial layer of a liver.
[Fig. 8B] Fig. 8B is a schematic view showing a case where the structured pattern is a straight pattern.
[Fig. 8C] Fig. 8C is a schematic view showing a case where the structured pattern is a concentric pattern.
[Fig. 9] Fig. 9 is a schematic view showing an example in which the density of the structured pattern in a part assumed as a boundary of an organ is increased.
[Fig. 10] Fig. 10 is a schematic view showing a constantly uniformly sparse structured pattern.
[Fig. 11] Fig. 11 is a schematic view showing a technique of separating the ranging image and the observation image in a case of performing irradiation with a constant structured pattern as shown in Fig. 10. Mode(s) for Carrying Out the Invention

Hereinafter, a suitable embodiment of the present disclosure will be described in detail with reference to the accompanying drawings. It should be noted that in this specification and drawings, constituent elements having substantially the same functional configurations are denoted by the same reference symbols and overlapping description thereof will be omitted.

It should be noted that description will be given in the following order.
1. Configuration Example of System
2. Configuration of Leading End of Rigid Scope
3. Regarding Specific Processing by System of Embodiment
4. Variations of Structured Pattern

### 1. Configuration Example of System

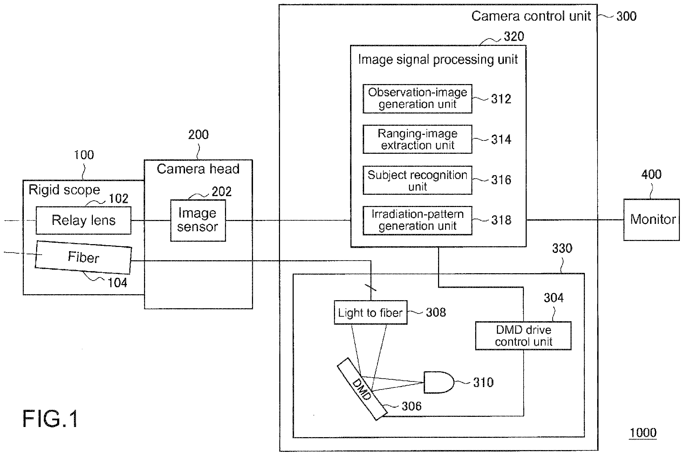

Fig. 1 is a schematic view showing a configuration of a system 1000 according to an embodiment of the present disclosure. As shown in Fig. 1, the system 1000 includes a rigid scope 100, a camera head 200, a camera control unit 300, and a monitor 400. The rigid scope 100 includes a relay lens 102 and a fiber 104. The camera head 200 includes an image sensor (imaging device) 202. The image sensor 202 is constituted by a CMOS sensor or the like. It should be noted that the rigid scope 100 is illustrated by an example in this embodiment, but this embodiment is also applicable to another endoscope such as a flexible scope.

The fiber 104 causes illumination light to propagate within the rigid scope 100 to irradiate a subject with light. When the subject is irradiated with light from the fiber 104, reflected light from the subject reaches an imaging surface of the image sensor 202 via the relay lens 102, and an image signal is acquired. As shown in Fig. 1, an optical axis of the fiber 104 and that of the relay lens 102 are not disposed in parallel. The fiber 104 and the relay lens 102 are disposed to form a predetermined angle therebetween such that a position of a structured pattern 510 to be described later changes depending on the shape of the subject when the subject is irradiated with the structured pattern 510 from the fiber 104. It should be noted that when a distance from the rigid scope 100 to the subject is small, the fiber 104 and the relay lens 102 may be disposed in parallel.

The camera control unit 300 includes an image signal processing unit 320, a DMD drive control unit 304, a DMD 306, a fiber 308, and a light source 310. The DMD drive control unit 304 and the DMD 306 constitute a light source of a DLP method (irradiation-pattern output unit 330) that is capable of appropriately switching between observation illumination and structured-pattern illumination. As an example, the resolution of the DMD 306 is approximately 2560*1600 pixels, and the diameter of the fiber 308 is approximately 966 pixels. Thus, a condenser lens is provided between the DMD 306 and the fiber 308. Because the DLP method has characteristics of a short response time and a bright light source, it is suitable to apply the DLP method to this embodiment. Meanwhile, if another method such as a transmissive or reflective liquid crystal method satisfies the requirements of the response time and brightness, such a method is also applicable to this embodiment.

The image signal processing unit 320 includes an observation-image generation unit 312, a ranging-image extraction unit 314, a subject recognition unit 316, and an irradiation-pattern generation unit 318.

### 2. Configuration of Leading End of Rigid Scope

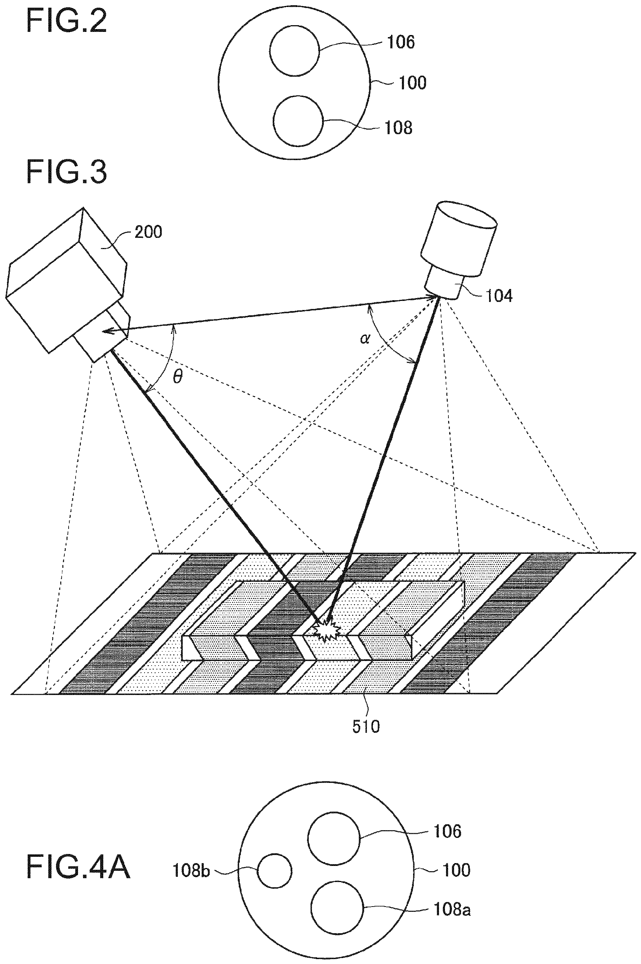

Fig. 2 is a schematic view showing a leading end of the rigid scope 100. As shown in Fig. 2, a camera window (acquisition unit) 106 that propagates light to the relay lens 102 and an illumination window (irradiation unit) 108 that irradiates a subject with light from the fiber 104 are provided to the leading end of the rigid scope 100. The illumination window (irradiation unit) 108 irradiates the subject with illumination light and also irradiates the subject with a structured pattern for acquiring depth information of the subject. The camera window (acquisition unit) 106 acquires reflected light of the light emitted through the illumination window (irradiation unit) 108.

When the rigid scope 100 is inserted into the inside of a human body and a subject such as an internal organ is imaged, there is a demand for acquiring distance information of the subject. In this embodiment, as shown in Fig. 3, the subject is irradiated with the structured pattern 510 from the fiber 104, a displacement of the structured pattern 510 that corresponds to the irregularities of the subject is detected on the basis of a change of the structured pattern 510 on the subject, and the structure of the subject (distance information) is estimated. Such a technique is known as a structured light method. Meanwhile, a captured image of the structured pattern 510 (structured image) includes a geometric pattern superimposed on the subject, and thus it is not appropriate as an observation image.

Meanwhile, in a case where both of observation illumination for a normal observation and illumination independently used for structured light are performed, as shown in Fig. 4A, both of a normal illumination window 108a and an illumination window 108b for structured light are provided to the leading end of the rigid scope 100. In this case, the distance information can be obtained from the structured pattern 510. However, because of an increase in number of illumination windows as compared to Fig. 2, the diameter of the leading end of the rigid scope 100 becomes difficult to reduce. Furthermore, it is necessary to switch between the normal illumination and the illumination of the structured pattern 510 in a time division manner, and in order to acquire the structured image, the frame rate of a normal image is reduced by half.

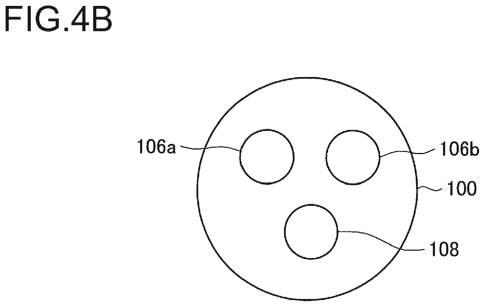

Further, in a case where the camera head 200 is configured to be capable of stereoscopic vision, as shown in Fig. 4B, two camera windows 106a and 106b are provided to the leading end of the rigid scope 100 so as to acquire right and left images. In this case, the distance information of the subject can be obtained from the parallax of the right and left images. However, because of an increase in number of camera windows as compared to Fig. 2, the diameter of the leading end of the rigid scope 100 also becomes difficult to reduce.

In this embodiment, a single illumination window 108 is provided to the leading end of the rigid scope 100, and the observation illumination and the illumination of the structured pattern 510 are switched on the camera control unit 300 side to irradiate the subject with the illumination. Subsequently, signal processing is performed such that an observation image and a structured image are separated from each other from the image acquired by the image sensor 202. Accordingly, the distance information can be obtained from the structured pattern 510, and since it is unnecessary to separately provide an illumination window for irradiating the subject with the structured pattern 510, reducing the diameter of the leading end of the rigid scope 100 can be achieved.

### 3. Regarding Specific Processing by System of Embodiment

Hereinafter, switching of the structured pattern 510 will be described. In this embodiment, the structured pattern 510 is switched in chronological order. More specifically, the subject is irradiated with an irradiation pattern 500, which is obtained by inverting the phases of the structured pattern 510 and the observation illumination on a frame-by-frame basis, and the subject is imaged by the image sensor 202. A captured image 530 obtained by the imaging includes structured pixels 532 in which a position of the structured pattern 510 changes depending on the shape of the subject. The image signal processing unit 320 extracts a ranging image 540 obtained when the structured pixels 532 are separated from the captured image 530, and also generates an observation image 550 that does not include the structured pixels 532.

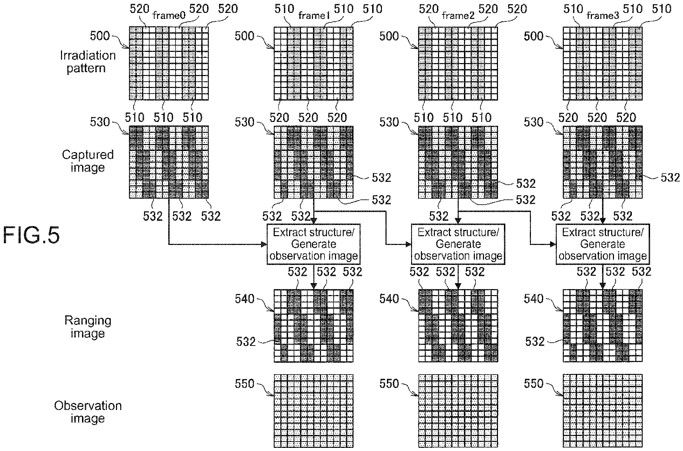

Fig. 5 is a schematic view showing the irradiation patterns 500 in this embodiment. As shown in the figures of "irradiation pattern" at the uppermost low of Fig. 5, the subject is irradiated with, as the irradiation patterns 500, patterns obtained by inverting the phases of the structured pattern 510 and observation illumination 520 on a frame-by-frame basis (frames 0 to 3). Subsequently, images are captured by the image sensor 202 in the respective frames (frames 0 to 3).

Fig. 5 shows captured images 530 in the respective frames below the irradiation patterns 500. As shown in Fig. 5, the captured image 530 includes the structured pixels 532 that are imaged together with distorted structured patterns 510 along the shape of the subject. Subsequently, the structured pixels 532 and the observation image 550 are separated from the captured images 530 of the frame 0 and the frame 1. Similarly, the structured pixels 532 and the observation image 550 are separated from the captured images 530 of the frame 1 and the frame 2. The ranging image 540 is an image obtained when the structured pixels 532 are separated.

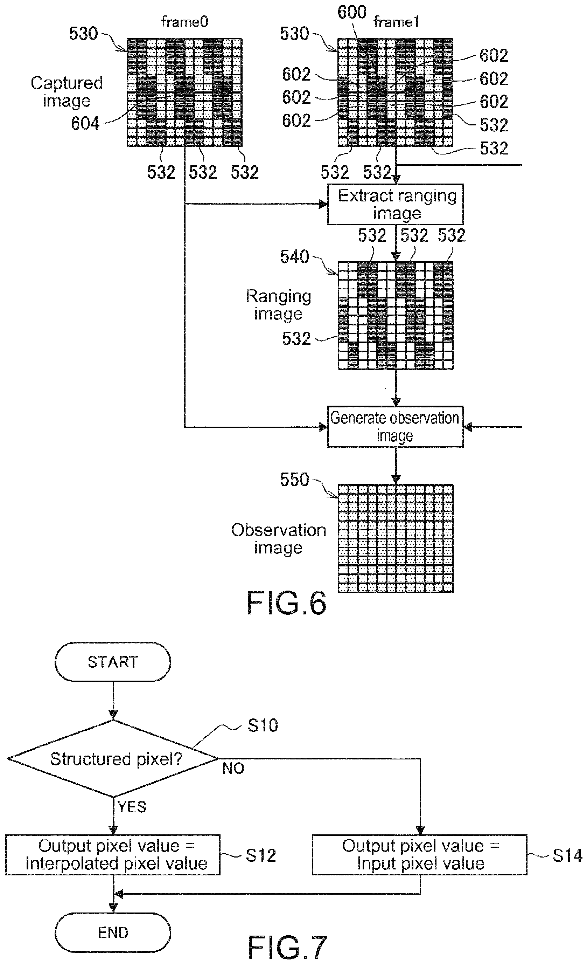

Fig. 6 is a schematic view showing a technique of separating the ranging image 540 and the observation image 550 from the captured images 530. Here, description will be given on a case of separating a ranging image 540 derived from the structured pattern 510 and an observation image 550 from the captured images 530 of the frame 0 and the frame 1. As shown in Fig. 6, first, a ranging image 540 is extracted from the captured image 530 of the frame 0 and the captured image 530 of the frame 1. Next, an observation image 550 is generated from the captured image 530 of the frame 0, the captured image 530 of the frame 1, and the ranging image 540.

Fig. 7 is a flowchart showing processing of separating the ranging image 540 and the observation image 550 on the basis of Fig. 6. The processing of Fig. 7 is performed on pixel values of the respective pixels that are input to the image signal processing unit 320. First, in Step S10, whether an arbitrary pixel, which is input to the image signal processing unit 320, is a pixel of the structured pattern 510 (structured pixel 532) or not is determined. Subsequently, as a result of the determination of Step S10, if the arbitrary pixel is the pixel of the structured pattern 510, the processing proceeds to Step S12, and an interpolated pixel value is output as an output pixel value. Meanwhile, as a result of the determination of Step S10, if the arbitrary pixel is not the pixel of the structured pattern 510, the processing proceeds to Step S14, and an input pixel value is output as an output pixel value.

In Step S10, determination on whether a pixel is the structured pixel 532 or not can be performed on the basis of a color of that pixel. In a case where the color of that pixel is a color that does not normally occur in a human body (e.g., blue), the pixel can be determined to be the structured pixel 532. Further, determination on whether a pixel is the structured pixel 532 or not can also be performed on the basis of a result of the determination of the previous frame. As described above, since the phases of the structured pattern 510 and the observation illumination 520 are inverted on a frame-by-frame basis, the pixel that is not the structured pixel 532 in the previous frame can be determined to be the structured pixel 532 in the current frame.

In Step S12, calculation of the interpolated pixel value is performed by the following methods. In a first method, in the pixels of the input frame, a value of a peripheral pixel that is not the structured pixel 532 is assumed as an interpolated pixel value. For example, a pixel value of a pixel 600 in the frame 1 of Fig. 6 is obtained by performing interpolation using pixel values of pixels 602, which are peripheral pixels but are not the pixels of the structured pattern 510. In the interpolation, general techniques such as distributing pixel values depending on distances from the respective pixel 602 and averaging those pixel values can be used. In a second method, a pixel value of a pixel having the same phase in the captured image 530 one frame before is used. For example, a pixel value of a pixel 604 in the frame 0 one frame before is used as the pixel value of the pixel 600 in the frame 1 of Fig. 6. If a large change in pixel value is not found between the frames, the pixel value of the pixel having the same phase in the captured image 530 one frame before can be used. In a third method, a pixel having the same phase in an observation synthetic image one frame before, and the like are used to generate the interpolated pixel value. In this case, if the pixel of the frame one frame before is the structured pixel 532, pixels that interpolate that structured pixel 532 are not the structured pixels 532. Thus, the structured pixel 532 of the current frame is interpolated by using the pixels that interpolate the structured pixel 532 in the frame one frame before.

In order to perform the processing as described above, the observation-image generation unit 312 of the image signal processing unit 320 separates the structured pixels 532 of the structured pattern 510 from the captured image 530 and generates an observation image 550. The ranging-image extraction unit 314 extracts the structured pixels 532 of the structured pattern 510 from the captured image 530 and acquires a ranging image 540.

### 4. Variations of Structured Pattern

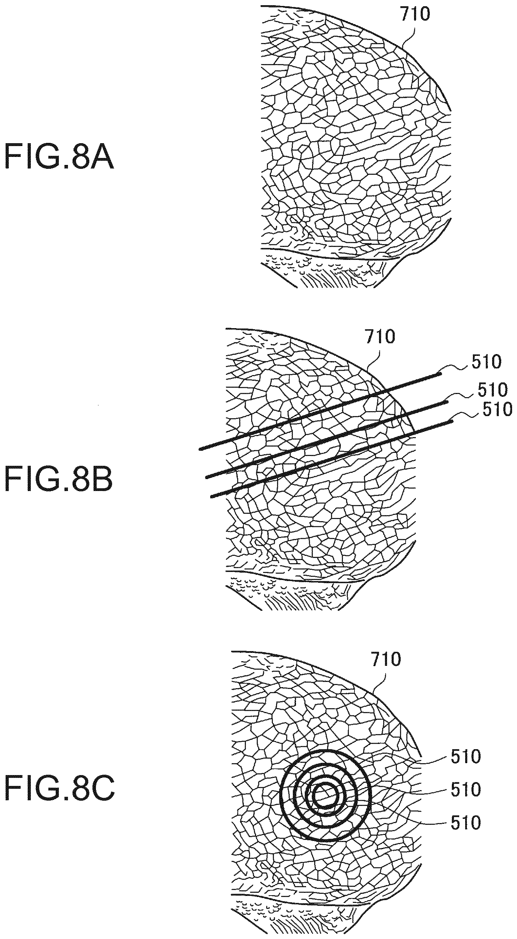

Regarding the structured pattern 510, the following variations are conceivable. In a first variation, the structured pattern 510 is a spatially uniform geometric pattern, and an organ 700 is irradiated with the structured pattern 510. In a second variation, as shown in Figs. 8A to 8C, the texture of an organ is recognized, and a pattern with a shape largely different from that texture is provided. Figs. 8A to 8C show a case where the organ is a liver 710 as an example. As shown in Fig. 8A, a superficial layer of the liver 710 has a characteristic crosshatch pattern, and thus this pattern and the structured pattern 510 are prevented from coinciding with each other. Fig. 8B shows a case where the structured pattern 510 is a straight pattern. In a case where the structured pattern 510 is a straight pattern, the structured pattern 510 may coincide with the crosshatch pattern of the superficial layer of the liver 710 and fail to detect the ranging image 540 (structured pixels 532). Fig. 8C shows a case where the structured pattern 510 is a concentric pattern. When the structured pattern 510 is a concentric pattern, a probability at which the crosshatch pattern of the surface of the liver 710 and the structured pattern 510 coincide with each other can be reduced, and a detection rate of the structured pixel 532 can be improved. By the above way, an incorrect recognition of the shape, which results from the texture of the organ, can be reliably suppressed.

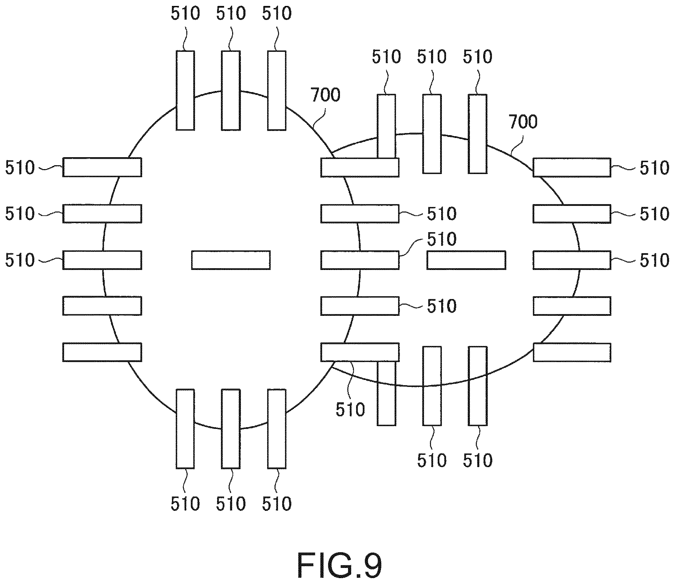

In a third variation, as shown in Fig. 9, the density of the structured pattern 510 in a part assumed as a boundary of the organ 700 is increased. The boundary of the organ 700 is acquired in advance by image processing (edge detection or the like), and the structured pattern 510 is disposed so as to have a high density in a region corresponding to the boundary. Accordingly, the shape with the depth at the boundary part of the organ 700 can be acquired highly accurately.

Thus, the subject recognition unit 316 of the image signal processing unit 320 recognizes the subject from the captured image 530. The irradiation-pattern generation unit 318 adaptively disposes the structured pattern 510 on the basis of a result of the recognition of the subject by the subject recognition unit 316 and generates the irradiation pattern 500. More specifically, the subject recognition unit 316 recognizes the outline of the subject from the captured image 530, and the irradiation-pattern generation unit 318 disposes the structured pattern 510 at a position of the outline and generates the irradiation pattern 500. Further, the subject recognition unit 316 recognizes a pattern of the surface of the subject from the captured image 530, and the irradiation-pattern generation unit 318 disposes the structured pattern 510 so as not to coincide with the pattern and generates the irradiation pattern 500. Information of the irradiation pattern 500 is transmitted to the irradiation-pattern output unit 330 constituted by the DMD drive control unit 304 and the DMD 306, and the subject is irradiated with the irradiation pattern 500 after passing through the fibers 104 and 308.

Further, regarding the color of the structured pattern 510, the following variations are assumed. In a first variation, the color of the structured pattern 510 is set to a plain color. For example, the color is set to blue having a small probability of occurrence in a living body (having small spectral distribution). Accordingly, in Step S10 of Fig. 7, the structured pattern 510 and the observation image 550 can be reliably separated from each other. In a second variation, the color of the structured pattern 510 is adaptively switched according to the color of the organ 700 of the subject. For example, in a case where the subject is an organ, the structured pattern 510 is set to blue, and in a case where the subject is forceps, the structured pattern 510 is set to yellow, thus improving separation between the pixels of the ranging image 540 and the observation image 550. As a third variation, it is also possible to add a light source of infrared rays (IR) and perform irradiation with the structured pattern 510 by the infrared rays. Using the infrared rays allows the ranging image 540 and the observation image 550 to be reliably separated from each other.

After the ranging image 540 and the observation image 550 are separated from each other, depth information is estimated from the ranging image 540. The estimation of the depth information can be performed by a well-known structured light method.

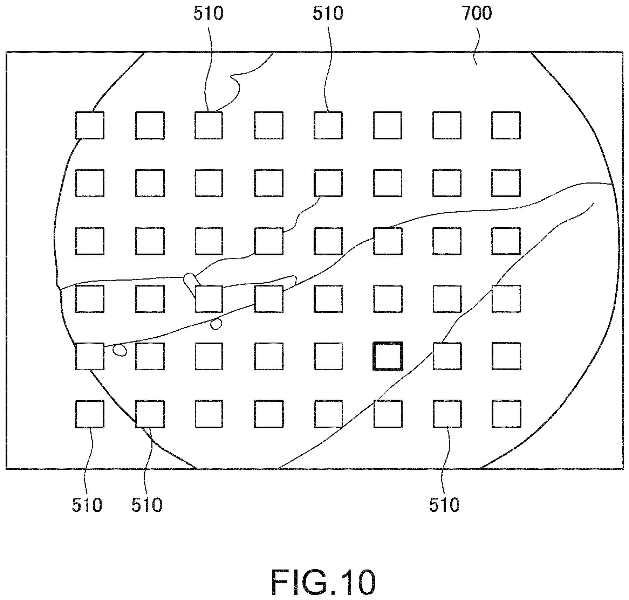

Next, description will be given on an example of performing irradiation with a constantly uniformly sparse structured pattern 510. In Fig. 5, the phases of the structured pattern 510 and the observation illumination 520 are inverted on a frame-by-frame basis. However, here, irradiation with a structured pattern 510 shown in Fig. 10 is constantly performed. Fig. 10 is a schematic view showing a constantly uniformly sparse structured pattern 510. In Fig. 10, irradiation with a blue structured pattern 510 is performed uniformly and sparsely in units of pixels. The structured pattern 510 is constituted by one pixel unit or a dot unit including a plurality of pixels (e.g., approximately four pixels).

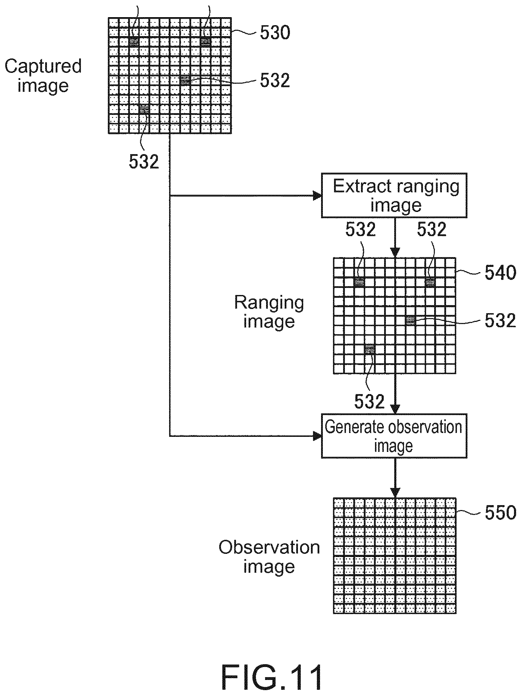

Fig. 11 is a schematic view showing a technique of separating the ranging image 540 and the observation image 550 in a case of performing irradiation with the constant structured pattern 510 as shown in Fig. 10. As shown in Fig. 11, the ranging image 540 is extracted from the captured image 530 in an arbitrary frame. The extraction of the ranging image 540 can be performed on the basis of the color of the captured image 530. When the color of the structured pattern 510 is set to a color that does not occur in a human body, such as blue, and if the captured image 530 has a pixel that does not occur in a human body, such a pixel can be determined to be the structured pixel 532. If the ranging image 540 can be extracted, the structured pixels 532 corresponding to the structured pattern 510 in the ranging image 540 are interpolated by using surrounding pixels, and the observation image 550 can thus be obtained.

The basic processing of generating the observation image 550 is similar to that of Fig. 7. In other words, in Step S10, whether an arbitrary pixel of the captured image 530 is a pixel of the structured pattern 510 (structured pixel 532) or not is determined. Subsequently, as a result of the determination of Step S10, if the arbitrary pixel is the pixel of the structured pattern 510, the processing proceeds to Step S12, and an interpolated pixel value is output as an output pixel value. Meanwhile, as a result of the determination of Step S10, if the arbitrary pixel is not the pixel of the structured pattern 510, the processing proceeds to Step S14, and an input pixel value is set as an output pixel value.

A ranging mode by the irradiation with the structured pattern 510 can be adaptively switched between on and off. For example, in a case where large motion is detected in the observation image 550, the ranging mode is turned off in order to avoid causing negative effects caused by an erroneous detection. Accordingly, the irradiation with the structured pattern 510 is not performed. Further, in a case where mist occurs in the subject, an erroneous detection of ranging may be performed due to the reflection of light or the like, and thus the ranging mode is turned off and the irradiation with the structured pattern 510 is not performed. Accordingly, it is possible to avoid causing negative effects caused by an erroneous detection. Further, it may also be possible to normally turn the ranging mode off, and only in a scene where a precise procedure such as suturing is necessary, to turn the ranging mode on to perform the irradiation with the structured pattern 510.

Hereinabove, a suitable embodiment of the present disclosure has been described in details with reference to the accompanying drawings, while the technical range of the present disclosure is not limited to the above examples. It is obvious that a person having ordinary skill in the technical field of the present disclosure could arrive at various alterations or modifications within the technical ideas described in the scope of claims, and it should be understood that they will naturally come under the technical scope of the present disclosure.

The effects described in this specification are merely illustrative or exemplified effects, and are not limitative. That is, with or in the place of the above effects, the technology according to the present disclosure may achieve other effects that are clear to those skilled in the art on the basis of the description of this specification.

It should be noted that the following configurations also come under the technical range of the present disclosure.
(1) An endoscope apparatus, including:
   an irradiation unit that irradiates a subject with illumination light and with a structured pattern for acquiring depth information of the subject; and
   an acquisition unit that acquires reflected light of the light emitted by the irradiation unit.
(2) The endoscope apparatus according to (1), further including:
   an imaging device that captures an image of the reflected light; and
   an image signal processing unit that processes an image signal of a captured image captured by the imaging device, in which
   the image signal processing unit includes an observation-image generation unit that separates structured pixels of the structured pattern from the captured image and generates an observation image.
(3) The endoscope apparatus according to (2), in which
   the image signal processing unit includes a ranging-image extraction unit that extracts the structured pixels of the structured pattern from the captured image and acquires a ranging image.
(4) The endoscope apparatus according to (2), in which
   the observation-image generation unit interpolates image information of pixels, from which the structured pixels are separated, and generates the observation image.
(5) The endoscope apparatus according to (4), in which
   the observation-image generation unit interpolates the image information of the pixels, from which the structured pixels are separated, by using image information of a peripheral pixel.
(6) The endoscope apparatus according to (4), in which
   the observation-image generation unit interpolates the image information of the pixels, from which the structured pixels are separated, by using image information of a corresponding pixel in a different frame.
(7) The endoscope apparatus according to (2), further including
   an irradiation-pattern output unit that outputs the illumination light and an irradiation pattern including the structured pattern to the irradiation unit.
(8) The endoscope apparatus according to (7), in which
   the irradiation-pattern output unit outputs the irradiation pattern by inverting pixels to which the structured pattern is output and pixels to which the structured pattern is not output on a frame-by-frame basis.
(9) The endoscope apparatus according to (7), in which
   the irradiation-pattern output unit sets pixels to which the structured pattern is output as constant pixels that do not change for each frame, and outputs the irradiation pattern.
(10) The endoscope apparatus according to (7), in which
   the irradiation-pattern output unit outputs the irradiation pattern including the structured pattern of a predetermined color.
(11) The endoscope apparatus according to (10), in which
   the predetermined color is a color that does not occur inside of a human body.
(12) The endoscope apparatus according to (11), in which
   the predetermined color includes blue or yellow.
(13) The endoscope apparatus according to (7), in which
   the image signal processing unit includes
   a subject recognition unit that recognizes the subject from the captured image, and
   an irradiation-pattern generation unit that disposes the structured pattern on the basis of a result of subject recognition by the subject recognition unit and generates the irradiation pattern.
(14) The endoscope apparatus according to (13), in which
   the subject recognition unit recognizes an outline of the subject from the captured image, and
   the irradiation-pattern generation unit disposes the structured pattern at a position of the outline and generates the irradiation pattern.
(15) The endoscope apparatus according to (13), in which
   the subject recognition unit recognizes a pattern of a surface of the subject from the captured image, and
   the irradiation-pattern generation unit disposes the structured pattern to avoid coinciding with the pattern and generates the irradiation pattern.
(16) An image generation method for an endoscope apparatus, the endoscope apparatus including an irradiation unit that irradiates a subject with illumination light and with a structured pattern for acquiring depth information of the subject, and an acquisition unit that acquires reflected light of the light emitted by the irradiation unit, the method including:
   capturing an image of the reflected light and acquiring a captured image; and
   separating structured pixels of the structured pattern from the captured image and generating an observation image.

### Reference Signs List

- 106: camera window
- 108: illumination window
- 312: observation-image generation unit
- 314: ranging-image extraction unit
- 316: subject recognition unit
- 318: irradiation-pattern generation unit
- 320: image signal processing unit
- 330: irradiation-pattern output unit

## Claims

1. An endoscope apparatus, comprising:
an irradiation unit that irradiates a subject with illumination light and with a structured pattern for acquiring depth information of the subject; and
an acquisition unit that acquires reflected light of the light emitted by the irradiation unit.

2. The endoscope apparatus according to claim 1, further comprising:
an imaging device that captures an image of the reflected light; and
an image signal processing unit that processes an image signal of a captured image captured by the imaging device, wherein
the image signal processing unit includes an observation-image generation unit that separates structured pixels of the structured pattern from the captured image and generates an observation image.

3. The endoscope apparatus according to claim 2, wherein
the image signal processing unit includes a ranging-image extraction unit that extracts the structured pixels of the structured pattern from the captured image and acquires a ranging image.

4. The endoscope apparatus according to claim 2, wherein
the observation-image generation unit interpolates image information of pixels, from which the structured pixels are separated, and generates the observation image.

5. The endoscope apparatus according to claim 4, wherein
the observation-image generation unit interpolates the image information of the pixels, from which the structured pixels are separated, by using image information of a peripheral pixel.

6. The endoscope apparatus according to claim 4, wherein
the observation-image generation unit interpolates the image information of the pixels, from which the structured pixels are separated, by using image information of a corresponding pixel in a different frame.

7. The endoscope apparatus according to claim 2, further comprising
an irradiation-pattern output unit that outputs the illumination light and an irradiation pattern including the structured pattern to the irradiation unit.

8. The endoscope apparatus according to claim 7, wherein
the irradiation-pattern output unit outputs the irradiation pattern by inverting pixels to which the structured pattern is output and pixels to which the structured pattern is not output on a frame-by-frame basis.

9. The endoscope apparatus according to claim 7, wherein
the irradiation-pattern output unit sets pixels to which the structured pattern is output as constant pixels that do not change for each frame, and outputs the irradiation pattern.

10. The endoscope apparatus according to claim 7, wherein
the irradiation-pattern output unit outputs the irradiation pattern including the structured pattern of a predetermined color.

11. The endoscope apparatus according to claim 10, wherein
the predetermined color is a color that does not occur inside of a human body.

12. The endoscope apparatus according to claim 11, wherein
the predetermined color includes blue or yellow.

13. The endoscope apparatus according to claim 7, wherein
the image signal processing unit includes
a subject recognition unit that recognizes the subject from the captured image, and
an irradiation-pattern generation unit that disposes the structured pattern on the basis of a result of subject recognition by the subject recognition unit and generates the irradiation pattern.

14. The endoscope apparatus according to claim 13, wherein
the subject recognition unit recognizes an outline of the subject from the captured image, and
the irradiation-pattern generation unit disposes the structured pattern at a position of the outline and generates the irradiation pattern.

15. The endoscope apparatus according to claim 13, wherein
the subject recognition unit recognizes a pattern of a surface of the subject from the captured image, and
the irradiation-pattern generation unit disposes the structured pattern to avoid coinciding with the pattern and generates the irradiation pattern.

16. An image generation method for an endoscope apparatus, the endoscope apparatus including an irradiation unit that irradiates a subject with illumination light and with a structured pattern for acquiring depth information of the subject, and an acquisition unit that acquires reflected light of the light emitted by the irradiation unit, the method comprising:
capturing an image of the reflected light and acquiring a captured image; and
separating structured pixels of the structured pattern from the captured image and generating an observation image.
